# EUROPEAN PATENT SPECIFICATION

(11) **EP 0 482 243 B1**
(45) Date of publication and mention of the grant of the patent: **05.04.1995**
(21) Application number: 90120519.5
(22) Date of filing: 26.10.1990
(51) Int. Cl.: A61B 5/0444, H05K 5/02

(54) **Medical monitor**
Medizinisches Überwachungsgerät
Dispositif de surveillance médical

(43) Date of publication of application: 29.04.1992
(73) Proprietor: Hewlett-Packard GmbH, D-71004 Böblingen (DE)
(72) Inventor: Rogler, Dietrich, W-7240 Horb 1 (DE)
(74) Representative: Kurz, Peter

(56) References cited:
- EP-A- 0 340 444
- DE-A- 3 542 871
- DE-U- 6 948 919
- DE-U- 7 506 668
- US-A- 3 621 844
- US-A- 4 866 215

## Description

The present invention relates to a medical monitor, in particular a fetal monitor, with fluid-repellent characteristics in a vertical mode of operation, including a casing which contains electronic circuitry for receiving, processing, displaying and/or recording of signals representative of physiological parameters of a human being, said casing comprising a front panel.

Medical monitors are used in a variety of clinical applications, e.g. in the operating room, in the anesthesia or recovery room, in the intensive care unit or in the delivery room. They are, for example, used to monitor the electrocardiogram of a patient, its respiration, its body temperature, various blood gases and so on, in order to allow a clinically meaningful evaluation of the patient's condition. Monitors of this type usually comprise a box or casing containing the printed circuit boards with the electronic circuitry necessary to process, filter etc. the physiological signals received from a sensor or transducer connected with the medical monitor. The output is then either displayed in numerical form or as a wave, e.g. on a cathode ray tube (CRT) or a 7-segment display. In addition, or as an alternative, the output may also be recorded on a printer. It may further be fed to a central station or the like.

For the purpose of interconnection with a central station, other monitors, recorders etc., most medical monitors are additionally equipped with jacks located at the rear panel of the casing. Examples of such connections are:
a) a digital system connector, i.e. a connection for exchanging digital data such as patient waves, trends, alarm messages etc., with a central station;
b) a digital or analog output for feeding signals, waves etc. to another monitor for display, or to a recorder;
c) digital or analog inputs, e.g. for receiving signals from another device, for the purpose of displaying, printing or recording;
d) an output for feeding digital or analog signals to a slave display, or via communication lines - such as a telephone link - to a remote place, e.g. the physician's home.

The above list contains only some examples where a connection of a medical monitor with another device is necessary. Other kinds of connections may be required as well.

In the specific embodiment described herein, the invention relates to a fetal monitor. A fetal monitor measures and records the fetal beat-to-beat heart rate (FHR) and the uterus activity (toco) simultaneously, which allows an exact determination of the fetal condition. The fetal beat-to-beat heart rate may be obtained from an electrocardiogram signal, e.g. via a spiral electrode screwed into the fetal epidermis, or by the Doppler shift of an ultrasound signal (the moving parts of the fetal heart, i.e. its valves and walls, cause a Doppler shift in the reflected ultrasound wave). If appropriate, the maternal electrocardiogram may be recorded as well.

Although the specific embodiment described herein is a fetal monitor - in fact, the present invention was made during the design process of such a fetal monitor -, the following description shows that the invention may be applied to other kinds of medical monitors as well.

A serious problem in the hospital, but also in the private practice of a physician, is simply the available space for all kinds of monitoring devices. There are several clinical applications, e.g. the operating room, where multiple physiological parameters of a patient have to be monitored. One has therefore already tried to combine several medical monitoring devices in multi-parameter monitors, or to incorporate various monitors in a cabinet. In the private practice of a physician, this does usually not help; although only one or very few medical parameters are recorded there, the available space is even considerably smaller than in a hospital application. This is particularly true in the case of an antepartum monitor, i.e. a fetal monitor used for screening measurements during gestation. However, even in hospital applications, the solutions of a cabinet or a combined monitor are not always adequate.

It was therefore a basic idea of the inventor in the present case to provide a medical monitor which cannot only be operated - as usual - in horizontal position, but also in vertical position. Therefore, it becomes possible to use the medical monitor not only on a desk, a shelf etc., but also to hang it on a wall or the like, and to operate it from there. This does not only save considerable space, but may also be useful in order to place the monitor close to a patient's bed. See also DE-U-6948919 which discloses an intensive care monitor intended for operation at a wall.

The feature of vertical operation is particularly suited for monitors which are light or have a relatively flat casing. Further, it is of particular value for monitors without a bulky CRT (although even monitors with a CRT could be operated in the vertical position).

If operated in vertical position, the medical monitor may be hung up on a wall in any convenient manner, e.g. using hooks, screws or the like.

When designing a medical monitor - here a fetal monitor - for operation in a vertical position, a problem encountered by the specific needs of a hospital, or, in general, medical environment was noted. This is that, in such environment, a monitor must be resistant against spray of water, disinfection fluids as well as spillage of these and other fluids. Operation of the monitor must not be impaired when a fluid is spilled over the monitor (in industrial practice, the required safety of the monitor is therefore usually tested in a "spillage test").

The requirement outlined above cannot be fulfilled if the connector jacks of the medical monitor are - as it is usual in the art - placed on the rear panel of the monitor, as the rear panels becomes the upper panel in the vertical position. The same is true for the power inlet. If one of these jacks were placed at the rear panel - i.e. the upper panel in the vertical position -, a fluid spilled over the monitor would penetrate into its casing, so that monitor malfunction could not be excluded.

It is therefore a major objective of the present invention to provide a medical monitor which may not only be operated in the horizontal position, but also in the vertical position, whereby safe operation is particularly ensured in the vertical position when a fluid is spilled or sprayed over the monitor.

According to the invention, this object is solved in a medical monitor of the kind described above by means of the following features:
a) the bottom side of said casing comprises a recess with at least three lateral walls,
b) at least one of said lateral walls is substantially parallel to and located opposite of said front panel, so that a normal vector on its surface is basically oriented in the same direction as a normal vector on the front panel, and
c) said one of said lateral walls comprises at least one jack for connection of an interface cable or the like.

The recess at the bottom site of the medical monitor is preferably rectangular, with its edges being substantially parallel to the outer edges of the monitor. However, other suitable geometric shapes like a triangle or a polygon may be used as well.

Such a recess comprises several surfaces. One is, of course, the bottom surface, i.e. a plane substantially parallel to the bottom surface of the monitor. The other surfaces are lateral or side walls which are substantially parallel to (or slightly inclined with respect to) the side walls of the monitor.

According to the invention, at least one of said lateral walls is substantially parallel to and located opposite of said front panel. That is, the normal on said specific lateral wall points basically in the same direction as the normal on the front panel. (It may also be inclined with respect to the front panel. The wording "basically oriented in the same direction" as used herein means the exactly parallel case as well as an inclined configuration). In the case of a rectangular configuration, said one of said lateral walls is opposite to the wall in the recess adjoining or being nearest to the front panel.

Said one of said lateral walls carries the connectors and/or jacks for connection of the medical monitor with other devices, such as system connectors, digital or analog inputs and outputs etc. However, it may not only carry the connectors and/or jacks for connection of interface cables, but also the connectors/jacks necessary for connection of a sensor or transducer, or for power connection. Still there are other advantageous solutions to position the power inlet and the patient connectors, e.g. to position the power outlet on a side wall of the casing, and the patient connectors on the front panel.

Therefore, the rear panel of the medical monitor, which becomes the upper panel when the monitor is operated in the vertical position, need not carry connectors or jacks. (However, a jack for earth connection of the patient, or the like, which cannot cause malfunction of the monitor, may still be placed on the rear panel). On the other hand, the connectors or jacks positioned on said one of said lateral walls in said recess point to the ground, i.e. are oriented downwards, if the monitor is operated in its vertical position. Therefore, no sprayed or spilled fluid may penetrate into the opening constituted by any connector or jack, so that, in turn, the medical monitor becomes fluid-proof. It may therefore be operated in the horizontal as well as in the vertical position, even in a medical environment.

An equipment enclosure with a cable management system incorporating a recess and a vertical wall is also disclosed in US 4,866,215. The vertical wall of this prior art device is oriented in a direction opposite to the front panel.

If the jacks are placed, as the present invention proposes, on a lateral wall of a recess on the bottom side of the medical monitor, the connectors of the interface cables or the like are contained, if connected, in said recess. In order to feed the cables to the outside, particularly if the monitor is operated in its horizontal position, various solutions are possible. In a preferred embodiment, the bottom side of the casing comprises, outside said recess, at least three - preferably four - base support plugs or studs. It is therefore easy to feed the interface cables to the outside using the space between the monitor and its support.

In another preferred embodiment, the bottom side of said casing comprises a second recess arranged between and connecting said one of said lateral walls and the rear panel of said casing. This feature makes it possible to feed the interface cables also to the rear side of the medical monitor while being kept in said second recess. This is of importance in order to keep the cables away from the person operating the monitor, or to reduce the visible numbers of cables leading to the monitor (in addition to the power cable and the patient connections). Last not least, keeping the interface cables away from the operator's field of vision is also a question of the optical appearance of the whole unit.

Advantageously, said second recess comprises a strain relieving device. This ensures that the interface cables are kept reliably in said second recess, particularly if the monitor is operated in its horizontal position. It also ensures that mechanical stress may not cause damage to any of the connections. Preferably, the strain relieving device consists of at least one tongue protruding from a side wall of the second recess, in particular a resilient tongue made of elastic material such as plastic material. The distance between said tongue and the bottom of the second recess is slightly smaller than the diameter of an interface cable, so that the cables pushed between the bottom of the second recess and the tongue are kept in this position, due to the elastic properties of the tongue. The tongue may also comprise an additional nose which prevents the interface cables from sliding out of their position between the tongue and the bottom of said second recess.

In an alternate embodiment, the tongue may be hinged to the casing of the monitor, i.e. devoted in said casing, whereby spring means force the tongue in its closed position. That is, the tongue may be clapped into an "open" and a "closed" position, wherein said spring means force it into the closed position (in the closed position, the tongue is in contact with the interface cables and keeps them in their relative position). The spring means may be any suitable spring known in the art, for example a leg spring or a spring clip.

In an advantageous and preferred embodiment, the medical monitor comprises a torus-like shoulder basically extending around said casing in parallel direction to the front panel, such that a part of a side wall of said torus-like shoulder adjoins said recess and forms said one of said lateral walls. The torus-like shoulder carries the displays, push buttons etc. necessary to operate the monitor. As it joins the first recess, its side wall is identical with the lateral wall of the first recess located opposite to said front panel. Therefore, said specific lateral wall is of greater depth than the other lateral walls. A particular advantage of this design is that, due to the increased depth, the available space for placing connectors or jacks is increased. In other words, the first recess may be of limited depth only, whereas the remaining space on said specific lateral wall is still sufficient to position several jacks or connectors thereon. In case such torus-like shoulder is provided, it may further be advantageous to form the second recess in said shoulder. Although the torus-like shoulder preferably surrounds the medical monitor, i.e. extends around the whole casing, it will be understood that this is not a necessary condition.

More features and advantages of the invention can be found in the claims and in the detailed description.

The invention will now be explained, by means of a non-limiting example, with reference to the accompanying drawings, in which
- Fig. 1: is a bottom view of a medical monitor according to the invention,
- Fig. 2: is a bottom view with a different arrangement of the interface cables,
- Fig. 3: depicts the medical monitor operated in vertical position,
- Fig. 4: depicts the monitor if operated in its horizontal position and
- Fig. 5: is another example of the vertical operating position, with the cables being fed in a different manner.

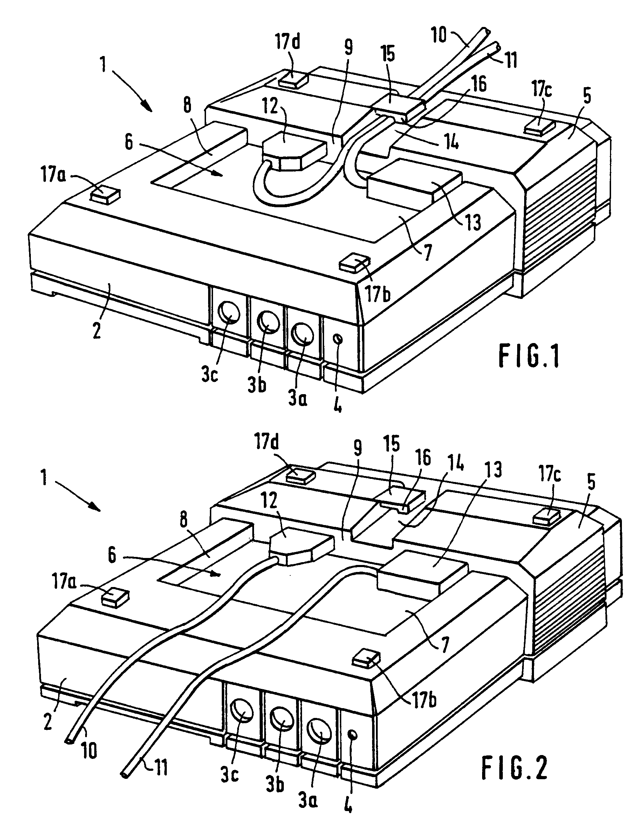

Fig. 1 depicts the bottom view of a fetal monitor 1 (which is an antepartum monitor here). A front panel 2 carries a connector 3a for connection of an ultrasound transducer (which is used to determine the fetal beat-to-beat heart rate). Connector 3b is provided for a toco (labor activity) transducer, and connector 3c provides a signal obtained from a second fetus (in case that twins are to be monitored). A fourth jack 4 is provided for connection of an external event marking device.

The casing of fetal monitor 1 further comprises a torus-like shoulder 5 extending around the casing and being manufactured as integral part of the casing. A recess 6 of rectangular shape is formed in the bottom of the monitor. It comprises a bottom surface or wall 7 and four lateral or side walls, two of which are visible in Fig. 1 and labelled as 8 and 9. The lateral wall next to front panel 2 is slightly inclined (not shown in Fig. 1).

Lateral wall 9 is parallel to front panel 2 and located opposite to it, i.e. faces the same direction. Its depth is greater than the depth of lateral wall 8, as it is also part of torus-like shoulder 5.

Lateral wall 9 further carries two jacks or connectors for connection of two interface cables 10 and 11, which in turn are equipped with connectors 12 and 13. Interface cables 10 and 11 are fed through a second recess 14 formed in torus-like shoulder 5. A tongue 15 is either pivoted in the casing or made of elastic material and keeps cables 10 and 11 in place. A nose 16 prevents the cables from sliding away.

The bottom side of monitor 1 comprises further, outside of recess 6, for support plugs or studs 17a to 17d.

In operation, interface cables 10 and 11 are either fed to the rear side of fetal monitor 1, as shown in Fig. 1, or to its front side, as Fig. 2 depicts. The plugs or studs 17a to 17d ensure, in the environment of Fig. 2, that the interface cables may be fed through the space between the fetal monitor and its support.

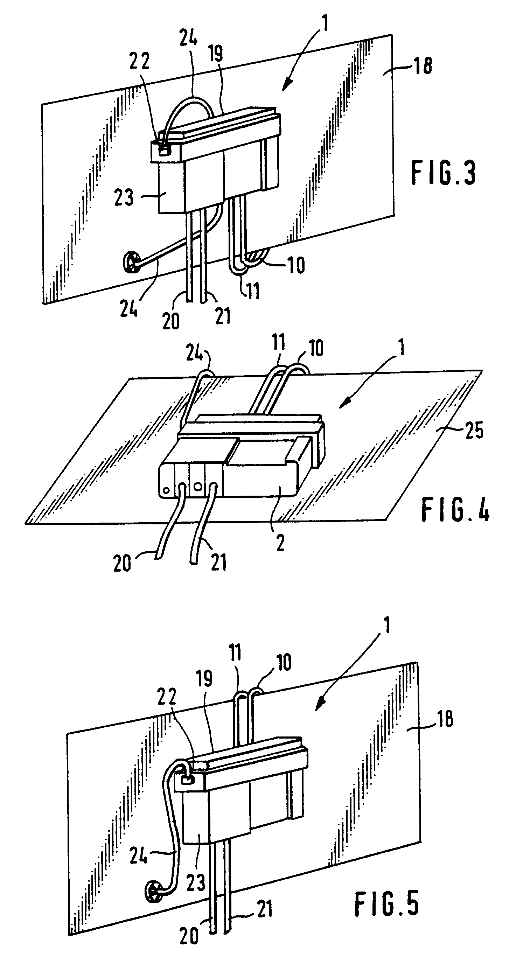

The schematics in Fig. 3 depicts fetal monitor 1 operated in vertical position. The monitor is fastened at a wall 18 in any convenient manner, e.g. using hooks, screws or the like. In this position rear panel 19 of the monitor becomes its top panel. As this panel does not carry any jacks or connectors, and as the interface connectors are directed downwards, the monitor is proof against strayed or spilled fluids.

The interface cables 10 and 11 extend in the direction of the front panel, as depicted in Fig. 2. Further, two patient cables 20 and 21 are connected. The jacks for these cables are also oriented downwards, so that no fluids may penetrate into the monitor. A power inlet 22 is arranged on a side wall 23 of the monitor, and a power cable 24 fed through recess 14 (Figs. 1,2) is connected with said power inlet.

Fig. 4 depicts fetal monitor 1 operated in its horizontal position. The monitor is placed on a horizontal support (e.g. a table) 25, wherein the torus-like shoulder 5 causes a slight inclination or tilt of the monitor, so that operation of the keys and reading of the displays (arranged on the top of the torus-like shoulder, but not drawn in Fig. 4) becomes easier. The arrangement of interface cables 10 and 11 is now the same as in Fig. 1, i.e. the interface cables are fed to the rear side of the monitor. The monitor is also safe against fluids in this horizontal position.

A further example, again with the monitor in the vertical position, is depicted in Fig. 5. Interface cables 10 and 11 are, in this environment, arranged as in Fig. 1, i.e. the interface cables are fed to the rear side of fetal monitor 1.

## Claims

1. Medical monitor (1), in particular fetal monitor, with fluid-repellent characteristics in a vertical mode of operation, including a casing which contains electronic circuitry for receiving, processing, displaying and/or recording of signals representative of physiological parameters of a human being, and which comprises a front panel (2), characterized in that
(1.1) the bottom side of said casing comprises a recess (6) with at least three lateral walls (8,9),
(1.2) at least one of said lateral walls (9) is substantially parallel to and located opposite of said front panel (2), so that a normal vector on its surface is basically oriented in the same direction as a normal vector on the front panel (2), and
(1.3) said one of said lateral walls (9) comprises at least one jack or connector for connection of an interface cable (10,11) or the like.

2. Medical monitor according to claim 1, characterized in that the bottom side of said casing comprises, outside said recess (6), at least three base support plugs (17a-17d).

3. Medical monitor according to claim 1 or 2, characterized in that the bottom side of said casing comprises a second recess (14) arranged between and connecting said one of said lateral walls (9) and the rear panel (19) of said casing.

4. Medical monitor according to claim 3, characterized in that said second recess (14) comprises a strain relieving device.

5. Medical monitor according to claim 4, characterized in that said strain relieving device consists of at least a tongue (15) protruding from a side wall of said second recess (14).

6. Medical monitor according to claim 5, characterized in that said tongue (15) is made of elastic material, preferably plastic material.

7. Medical monitor according to claim 5, characterized in that said tongue (15) is pivoted in said casing, whereby spring means force said tongue in its closed position.

8. Medical monitor according to any of the preceding claims, characterized by a torus-like shoulder (5) basically extending around said casing in parallel direction to said front panel (2), such that a part of a side wall of said torus-like shoulder (5) adjoins said recess (6) and forms said one of said lateral walls (9).

9. Medical monitor according to claims 3 and 8, characterized in that said second recess (14) is formed in said torus-like shoulder (5).

10. Medical monitor according to any of the preceding claims, characterized in that said front panel (2) comprises at least one jack (3a-3c) for the connection of a patient cable.

11. Medical monitor according to any of the preceding claims, characterized in that a power inlet jack (22) is arranged on a side wall (24) of said casing.

## Patentansprüche

1. Medizinischer Monitor (1), insbesondere ein Fötus-Monitor, mit flüssigkeitsabstoßenden Charakteristika in einem vertikalen Betriebsmodus, mit einem Gehäuse, das eine elektronische Schaltung zum Empfangen, Verarbeiten, Anzeigen und/oder Aufzeichnen von Signalen enthält, die physiologische Parameter eines Menschen darstellen, und der eine vordere Abdeckung (2) umfaßt,
dadurch gekennzeichnet, daß
(1.1) die Bodenseite des Gehäuses eine Ausnehmung (6) mit zumindest drei lateralen Wänden (8, 9) umfaßt,
(1.2) zumindest eine der lateralen Wände (9) im wesentlichen parallel und gegenüber der vorderen Abdeckung (2) angeordnet ist, so daß ein normaler Vektor auf deren Oberfläche grundsätzlich in dieselbe Richtung ausgerichtet ist wie ein normaler Vektor auf der vorderen Abdeckung (2), und
(1.3) eine der lateralen Wände (9) zumindest eine Buchse oder einen Verbinder zum Anschluß eines Schnittstellenkabels (10, 11) oder dergleichen umfaßt.

2. Medizinischer Monitor gemäß Anspruch 1, dadurch gekennzeichnet,
daß die Bodenseite des Gehäuses außerhalb der Ausnehmung (6) zumindest drei Basistragebolzen (17a - 17d) umfaßt.

3. Medizinischer Monitor gemäß Anspruch 1 oder 2, dadurch gekennzeichnet,
daß die Bodenseite des Gehäuses eine zweite Ausnehmung (14) umfaßt, die zwischen den lateralen Wänden (9) und der rückwärtigen Abdeckung (19) des Gehäuses angeordnet ist und diese verbindet.

4. Medizinischer Monitor gemäß Anspruch 3, dadurch gekennzeichnet,
daß die zweite Ausnehmung (14) ein Zugentlastungsgerät umfaßt.

5. Medizinischer Monitor gemäß Anspruch 4, dadurch gekennzeichnet,
daß das Zugentlastungsgerät aus zumindest einer Zunge (15) besteht, die von einer Seitenwand der zweiten Ausnehmung (14) hervorsteht.

6. Medizinischer Monitor gemäß Anspruch 5, dadurch gekennzeichnet,
daß die Zunge (15) aus einem elastischen Material, bevorzugterweise einem Kunststoffmaterial, hergestellt ist.

7. Medizinischer Monitor gemäß Anspruch 5, dadurch gekennzeichnet,
daß die Zunge (15) in dem Gehäuse drehbar ist, wobei eine Federeinrichtung die Zunge in ihre geschlossene Position zwingt.

8. Medizinischer Monitor gemäß einem der vorhergehenden Ansprüche, gekennzeichnet durch
einen Torus-artigen Absatz (5), der sich grundsätzlich um das Gehäuse in paralleler Richtung zu der vorderen Abdeckung (2) derart erstreckt, daß ein Teil der Seitenwand des Torus-artigen Absatzes (5) an die Ausnehmung (6) angrenzt und eine der lateralen Wände (9) bildet.

9. Medizinischer Monitor nach Anspruch 3 und 8, dadurch gekennzeichnet,
daß die zweite Ausnehmung (14) in dem Torus-artigen Absatz (5) gebildet ist.

10. Medizinischer Monitor nach einem der vorhergehenden Ansprüche, dadurch gekennzeichnet,
daß die vordere Abdeckung (2) zumindest eine Buchse (3a - 3c) für den Anschluß eines Patientenkabels umfaßt.

11. Medizinischer Monitor gemäß einem der vorhergehenden Ansprüche, dadurch gekennzeichnet,
daß eine Leistungseinlaßbuchse (22) an einer Seitenwand (24) des Gehäuses angeordnet ist.

## Revendications

1. Dispositif de surveillance médicale (1), plus particulièrement dispositif de surveillance foetale, présentant des caractéristiques de répulsion de fluide dans un mode de fonctionnement vertical, incluant un boîtier qui contient un circuit électronique permettant de recevoir, de traiter, d'afficher et/ou d'enregistrer des signaux représentatifs de paramètres physiologiques d'un être humain, lequel comprend un panneau avant (2),
caractérisé en ce que :
(1.1) le côté de fond dudit boîtier comprend un évidement (6) muni d'au moins trois parois latérales (8, 9),
(1.2) au moins l'une desdites parois latérales (9) est sensiblement parallèle audit panneau avant (2) et est positionnée de manière à lui faire face de telle sorte qu'un vecteur normal à sa surface soit fondamentalement orienté suivant la même direction qu'un vecteur normal au panneau avant (2) ; et
(1.3) ladite une desdites parois latérales (9) comprend au moins un jack ou connecteur permettant d'assurer la connexion d'un câble d'interface (10, 11) ou autre.

2. Dispositif de surveillance médicale selon la revendication 1, caractérisé en ce que le côté de fond dudit boîtier comprend, à l'extérieur dudit évidement (6), au moins trois plots de support de base (17a à 17d).

3. Dispositif de surveillance médicale selon la revendication 1 ou 2, caractérisé en ce que le côté de fond dudit boîtier comprend un second évidement (14) agencé entre ladite une desdites parois latérales (9) et le panneau arrière (19) dudit boîtier et connectant ceux-ci.

4. Dispositif de surveillance médicale selon la revendication 3, caractérisé en ce que ledit second évidement (14) comprend un dispositif de libération de contrainte.

5. Dispositif de surveillance médicale selon la revendication 4, caractérisé en ce que ledit dispositif de libération de contrainte est constitué par au moins une languette (15) qui fait saillie depuis une paroi latérale dudit second évidement (14).

6. Dispositif de surveillance médicale selon la revendication 5, caractérisé en ce que ladite languette (15) est réalisée en un matériau élastique, de préférence en une matière plastique.

7. Dispositif de surveillance médicale selon la revendication 5, caractérisé en ce que ladite languette (15) est pivotée dans ledit boîtier, un moyen de ressort poussant de manière forcée ladite languette dans sa position fermée.

8. Dispositif de surveillance médicale selon l'une quelconque des revendications précédentes, caractérisé par un épaulement en forme d'entourage (5) s'étendant fondamentalement autour dudit boîtier suivant une direction parallèle audit panneau avant (2) de telle sorte qu'une partie d'une paroi latérale dudit épaulement en forme d'entourage (5) rejoigne ledit évidement (6) et forme ladite une desdites parois latérales (9).

9. Dispositif de surveillance médicale selon les revendications 3 et 8, caractérisé en ce que ledit second évidement (14) est formé dans ledit épaulement en forme d'entourage (5).

10. Dispositif de surveillance médicale selon l'une quelconque des revendications précédentes, caractérisé en ce que ledit panneau avant (2) comprend au moins un jack (3a à 3c) pour la connexion d'un câble de patient.

11. Dispositif de surveillance médicale selon l'une quelconque des revendications précédentes, caractérisé en ce qu'un jack d'entrée d'alimentation (22) est agencé sur une paroi latérale (24) dudit boîtier.
